(19) Europäisches Patentamt
European Patent Office
Office européen des brevets

(11) Numéro de publication : **0 375 504 B1**

(12) # FASCICULE DE BREVET EUROPEEN

(45) Date de publication du fascicule du brevet : **25.03.92 Bulletin 92/13**

(51) Int. Cl.[5] : **A61K 31/415,** A61K 31/42

(21) Numéro de dépôt : **89403430.5**

(22) Date de dépôt : **12.12.89**

(54) **Application de benzamides substitués comme anxiolytiques et anti-psychotiques.**

(30) Priorité : **20.12.88 FR 8816764**

(43) Date de publication de la demande : **27.06.90 Bulletin 90/26**

(45) Mention de la délivrance du brevet : **25.03.92 Bulletin 92/13**

(84) Etats contractants désignés : **AT BE CH DE ES FR GB GR IT LI LU NL SE**

(56) Documents cités :
**EP-A- 0 236 646**
**EP-A- 0 295 350**
**US-A- 4 329 354**
**US-A- 4 333 945**

(73) Titulaire : **Laboratoires DELAGRANGE**
**1, Avenue Pierre-Brossolette**
**F-91380 Chilly-Mazarin (FR)**

(72) Inventeur : **Acher, Jacques**
**32, route de Saint Vrain**
**F-91760 Itteville (FR)**
Inventeur : **Monier, Jean-Claude**
**6, rue de la Sorbonne**
**F-91510 Lardy (FR)**
Inventeur : **Schmitt, Jean-Paul**
**13, rue Victor Hugo**
**91290 Arpajon (FR)**
Inventeur : **Costall, Brenda, Dr.**
**The Old Rectroy Addingham**
**Ilkey West Yorkshire (GB)**
Inventeur : **Naylor, Robert, Dr.**
**The Old Rectroy Addingham**
**Ilkey West Yorkshire (GB)**
Inventeur : **Gardaix-Luthereau, Renée**
**9, rue Guichard**
**F-94230 Cachan (FR)**

**EP 0 375 504 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une nouvelle application de benzamides substitués de formule (I) :

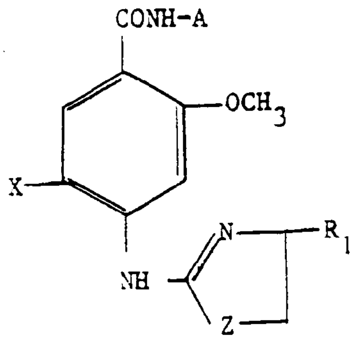

(I)

dans laquelle :

– A représente un groupe diéthylaminoéthyle ou un groupe de formule :

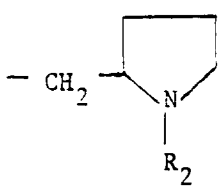

où $R_2$ est un groupe éthyle ou allyle

– $R_1$ représente un atome d'hydrogène ou un groupe méthyle,

– X représente un atome de chlore ou de brome,

– Z représente un groupe NH ou un atome d'oxygène, avec la condition suivante :

lorsque Z est un groupe NH et X un atome de chlore, $R_1$ est un groupe méthyle,

et de leurs sels pharmacologiquement acceptables.

L'invention concerne en particulier les composés suivants :

composé 1 : N-[2-(diéthylamino) éthyl] 2-méthoxy 4-[ (1H-4,5-dihydro 4-méthyl 2-imidazolyl) amino] 5-chloro benzamide.

composé 2 : N-(1-allyl 2-pyrrolidinylméthyl) 2-méthoxy 4-[ (1H-4,5-dihydro 4-méthyl 2-imidazolyl)amino] 5-chloro benzamide.

composé 3 : N-[ 2-(diéthylamino)éthyl] 2-méthoxy 4-[ (1H-4,5-dihydro 4-méthyl 2-imidazolyl)amino] 5-bromo benzamide.

composé 4 : N-(1-éthyl 2-pyrrolidinylméthyl)2-méthoxy 4-[ (1H-4,5-dihydro 4-méthyl 2-imidazolyl) amino] 5-bromo benzamide.

composé 5 : N-(1-éthyl 2-pyrrolidinylméthyl)2-méthoxy 4-[ (1H-4,5-dihydro 4-méthyl 2-imidazolyl)amino] 5-chloro benzamide.

composé 6 : N-[ 2-(diéthylamino)éthyl ] 2-méthoxy 4- [(1H-4,5-dihydro 2-imidazolyl)amino ]5-bromo benzamide.

composé 7 : N-[ 2-(diéthylamino)éthyl ] 2-méthoxy 4-[(4,5-dihydro 2-oxazolyl)amino] 5-chloro benzamide.

Les composés de l'invention étaient déjà connus comme activants du système nerveux central et comme antidépresseurs d'après la demande de brevet français n° 85-18829 (= EP-A-O 236 646).

Ces composés se distinguent, en tant que psychotropes, d'autres composés methoxy-benzamides par le fait qu'ils ne possèdent pas de propriétés anti-dopaminergiques. De ce fait, les composés selon l'invention ne sont pas des neuroleptiques au sens habituel du terme car ils ne se lient pas aux récepteurs de la dopamine D-1/D-2. Ils sont inactifs dans certains essais de comportement habituellement réalisés pour tester les neuroleptiques. Ces résultats ne pouvaient laisser supposer a priori que les composés selon l'invention soient tranquillisants et plus spécifiquement anxiolytiques.

Une étude très approfondie des composés selon l'invention a montré qu'ils possédaient des propriétés inattendues :

Anxiolytique

Les composés selon l'invention se révèlent puissamment anxiolytiques. Ceci est démontré par le test de la boîte à deux compartiments (sombre et éclairé) chez la souris.

Anti-psychotique (schizophrénie)

La théorie actuelle de la physiophathologie de la schizophrénie étant une activité dopaminergique accrue au niveau du mésolimbe, les médicaments actuellement utilisés comme anti-psychotiques sont des anti-dopaminergiques.
Bien que les composés de l'invention n'aient pas montré d'affinité pour les récepteurs de la dopamine comme il est connu pour les neuroleptiques, les tests comportementaux mis en application ont montré qu'ils supprimaient l'hyperactivité induite chez le rat, par administration intra-accumbens d'un agoniste de la dopamine : l'amphétamine, propriété prédictive d'une activité anti-psychotique.

Les tests ont été réalisés selon les protocoles suivants :

1. Test de la boîte à 2 compartiments chez la souris

Ce test est basé sur l'aversion naturelle des souris à l'égard de la lumière.

L'appareil utilisé est une boîte comprenant deux compartiments dont l'un est sombre (indiqué "compartiment noir" dans le tableau 1) et l'autre éclairé (indiqué "compartiment blanc" dans le tableau 1). Cet appareil est conçu pour laisser à l'animal le choix de séjourner dans l'un ou l'autre des compartiments. Dans des conditions normales, les souris évitent la lumière et restent surtout dans la partie sombre. Sous l'influence d'un anxiolytique, l'exploration du compartiment éclairé prédomine.

Le test consiste à déposer chaque souris au centre du compartiment éclairé et à observer le comportement de l'animal par système vidéo pendant 5 minutes. Le nombre de redressements exploratoires et le nombre de déplacements (franchissement de lignes tracées sur le plancher de chaque compartiment = passages) sont ensuite notés pour chaque compartiment : une augmentation du nombre de redressements exploratoires et de déplacements et du nombre de passages dans le compartiment éclairé est caractéristique de l'effet anxyolytique d'un composé.

Les composés de l'invention ont été injectés par voie sous-cutanée, à des doses variables, chaque dose étant administrée à un groupe de 5 souris. L'observation de chaque animal a eu lieu 40 minutes après administration du produit et les résultats ont été comparés aux résultats obtenus avec un groupe témoin n'ayant reçu qu'un véhicule inerte.

Les résultats obtenus sont rassemblés dans le tableau 1.

Il apparaît que les composés de l'invention ont une activité anxiolytique importante qui se manifeste par une augmentation significative du nombre des redressements exploratoires et des déplacements dans le compartiment éclairé, une augmentation du délai de passage du compartiment éclairé au compartiment sombre et une diminution du temps de séjour dans le compartiment sombre, à la dose de 0,1mg/kg par voie sous-cutanée. Les composés 1 et 2 manifestent même cette activité à partir de 0,01mg/kg et le composé 4 à partir de 0,0001mg/kg.

2. Hyperactivité induite chez le rat par perfusion intra-accumbens d'amphétamine

Ce test consiste à déterminer l'effet des composés de l'invention sur l'hyperactivité provoquée chez le rat par injection intra-accumbens d'amphétamine.

L'hyperactivité a été induite par injection intra-accumbens bilatérale de 20μg d'amphétamine.

Les composés de l'invention ont été administrés aux doses de 1 mg/kg et 10 mg/kg par voie sous-cutanée, 40 minutes avant l'injection d'amphétamine.

Pour chaque dose de produit à étudier, on a utilisé un groupe de 5 animaux. Un groupe, traité uniquement par l'amphétamine et un groupe de référence, n'ayant reçu qu'un véhicule inerte, étaient constitués de 10 animaux.

Immédiatement après l'injection d'amphétamine, les rats ont été placés dans des cages individuelles équipées de cellules photoélectriques. L'activité motrice, caractérisée par le nombre de déplacements dans ces cages, a été déterminée par le nombre d'interruptions d'un faisceau lumineux touchant ces cellules.

Les mesures ont été faites au cours des 80 minutes suivant l'injection d'amphétamine.

Les résultats, exprimés en nombre de déplacements par période de 10 minutes, sont rassemblés dans le tableau 2.

Il apparaît que les composés de l'invention, aux doses de 1 et 10 mg/kg par voie sous-cutanée, antagonisent l'hyperactivité induite par injection intra-accumbens d'amphétamine.

Cette action est hautement prédictive de propriétés antipsychotiques.

ACTIVITE ANXIOLYTIQUE

| Composé administré | compartiment blanc | | compartiment noir | | % temps dans compartiment noir | latence blanc → noir (sec) |
|---|---|---|---|---|---|---|
| | redressements/5mn | passages/5mn | redressements/5mn | passages/5mn | | |
| véhicule(1) | 27 | 37 | 67 | 84 | 56 | 9 |
| composé 1 | | | | | | |
| 0,01mg/kg | 57 | 77 | 16 | 37 | 36 | 19 |
| 0,1 mg/kg | 73 | 79 | 14 | 23 | 27 | 22 |
| composé 6 | | | | | | |
| 0,1mg/kg | 40 | 48 | 39 | 50 | 27 | 13 |
| composé 7 | | | | | | |
| 0,1mg/kg | 69 | 68 | 16 | 16 | 27 | 24 |
| véhicule (2) | 22 | 36 | 72 | 91 | 52 | 11 |
| composé 2 | | | | | | |
| 0,01mg/kg | 43 | 51 | 27 | 30 | 49 | 18 |
| 0,1 mg/kg | 83 | 80 | 20 | 25 | 29 | 20 |
| composé 3 | | | | | | |
| 0,1 mg/kg | 97 | 104 | 18 | 18 | 30 | 23 |
| composé 4 | | | | | | |
| 0,0001mg/kg | 73 | 82 | 19 | 21 | 27 | 23 |
| 0,01 mg/kg | 82 | 83 | 15 | 20 | 31 | 22 |
| 0,1 mg/kg | 78 | 99 | 16 | 24 | 34 | 38 |
| composé 5 | | | | | | |
| 1mg/kg | 65 | 89 | 14 | 19 | 32 | 23 |
| 10mg/kg | 68 | 80 | 15 | 16 | 32 | 22 |

TABLEAU 1

E.S.M. $< 12,6\%$
$n = 5$

$p < 0,05$ à $p < 0,001$ - tous les résultats portant sur les lots traités sont significatifs par rapport aux résultats des témoins.

ANTAGONISME DE L'HYPERACTIVITE INDUITE CHEZ LE RAT PAR INJECTION INTRA-ACCUMBENS D'AMPHETAMINE.

| Temps (min.) | Activité motrice (déplacements / 10 minutes) | | | | | |
|---|---|---|---|---|---|---|
| | amphétamine (20µg) | véhicule | composé 1 | | composé 4 | |
| | | | 1mg/kg | 10mg/kg | 1mg/kg | 10mg/kg |
| 10 | 31 ± 4 | 32 ± 3 | 49 ± 9 | 27 ± 4 | 34 ± 8 | 38 ± 7 |
| 20 | 48 ± 5 * | 29 ± 3 | 35 ± 3 | 27 ± 6 | 60 ± 13 * | 8 ± 1,4 + |
| 30 | 66 ± 7 * | 18 ± 3 | 45 ± 8 * | 13 ± 3 | 40 ± 10 * | 9 ± 3 |
| 40 | 59 ± 7 * | 9 ± 2 | 40 ± 8 * | 11 ± 2 | 38 ± 13 * | 11 ± 1,6 |
| 50 | 36 ± 5 * | 6 ± 1,4 | 11 ± 3 | 8 ± 2 | 37 ± 10 * | 5 ± 1 |
| 60 | 29 ± 4 * | 3 ± 1 | 12 ± 2 | | 16 ± 2 * | 2 ± 1 |
| 70 | 19 ± 3 | | 8 ± 1 | | 8 ± 3 | |
| 80 | 8 ± 3 | | | | 4 ± 2 | |

TABLEAU 2

* $p < 0,05$ à $p < 0,001$
\+ $p < 0,01$

Les composés de l'invention ont également fait l'objet d'une étude de toxicité aiguë par voie intraveineuse chez la souris mâle et la souris femelle, qui a permis de déterminer les doses léthales 50 (DL50).

Les résultats suivants ont été obtenus :

| Composé | $DL_{50}$ I.V. (mg/kg) | |
|---|---|---|
| | Souris mâle | Souris femelle |
| Composé 1 | 17,5 - 21,8 | 21,6 - 26,6 |
| Composé 2 | 35,1 - 50,2 | 35,1 - 50,2 |
| Composé 3 | 19,5 - 24,8 | 21,1 - 27,2 |
| Composé 4 | 13,1 - 18,3 | 15,7 - 20,7 |
| Composé 6 | 91 - 116 | 106 - 132 |

Les résultats obtenus sont compatibles avec une utilisation comme médicaments des composés de l'invention.

**Revendications**

1. Utilisation des composés de formule (I)

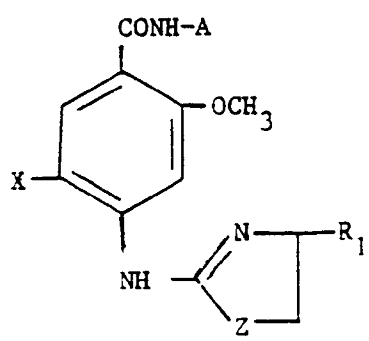

(I)

dans laquelle :

– A représente un groupe diéthylamino-éthyle ou un groupe de formule :

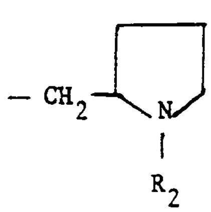

où $R_2$ est un groupe éthyle ou allyle,

– $R_1$ représente un atome d'hydrogène ou un groupe méthyle,

– X représente un atome de chlore ou de brome,

– Z représente un groupe NH ou un atome d'oxygène,

avec la condition suivante :

lorsque Z est un groupe NH et X un atome de chlore, $R_1$ est un groupe méthyle,

et de leurs sels pharmacologiquement acceptables, pour la préparation de médicaments utiles comme anxiolytiques et anti-psychotiques.

2. Utilisation, selon la revendication 1, du N-[ 2-(diéthylamino)éthyl] 2-méthoxy 4-[(1H-4,5-dihydro 4-méthyl 2-imidazolyl)amino] 5-chloro benzamide pour la préparation d'un médicament utile comme anxiolytique et anti-psychotique.

3. Utilisation, selon la revendication 1, du N-(1-allyl 2-pyrrolidinylméthyl) 2-méthoxy 4- [(1H-4,5-dihydro 4-méthyl 2-imidazolyl) amino] 5-chloro benzamide pour la préparation d'un médicament utile comme anxiolytique et anti-psychotique.

4. Utilisation, selon la revendication 1, du N-[2-(diéthylamino)éthyl] 2-méthoxy 4-[(1H-4,5-dihydro 4-méthyl 2-imidazolyl)amino] 5-bromo benzamide pour la préparation d'un médicament utile comme anxiolytique et anti-psychotique.

5. Utilisation, selon la revendication 1, du N-(1-éthyl 2-pyrrolidinylméthyl) 2-méthoxy 4-[(1H-4,5-dihydro 4-méthyl 2-imidazolyl)amino] 5-bromo benzamide pour la préparation d'un médicament utile comme anxiolytique et anti-psychotique.

6. Utilisation, selon la revendication 1, du N-[2-(diéthylamino)éthyl] 2-méthoxy 4-[(1H-4,5-dihydro 2-imidazolyl) amino] 5-bromo benzamide pour la préparation d'un médicament utile comme anxiolytique et anti-psychotique.

7. Utilisation, selon la revendication 1, du N-[2-(diéthylamino) éthyl] 2-méthoxy 4-[(4,5-dihydro 2-oxazolyl) amino] 5-chloro benzamide pour la préparation d'un médicament utile comme anxiolytique et anti-psychotique.

8. Utilisation, selon la revendication 1, du N-(1-éthyl 2-pyrrolidinyl-méthyl) 2-méthoxy 4-[(1H-4,5 - dihydro 4-méthyl 2-imidazolyl)amino] 5-chloro benzamide pour la préparation d'un médicament utile comme anxiolytique et anti-psychotique.

## Patentansprüche

1. Verwendung von Verbindungen der Formel (I)

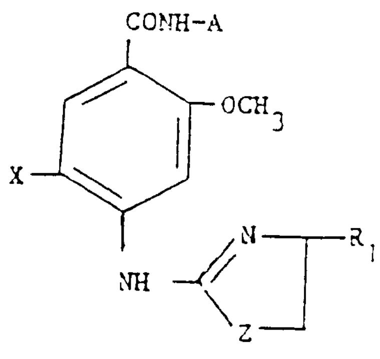

(I)

in der

– A eine Diethylaminoethylgruppe oder eine Gruppe der Formel

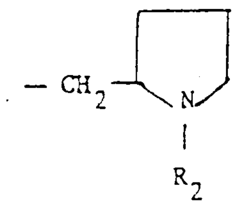

in der $R_2$ eine Ethyl- oder Allylgruppe ist,
- $R_1$ ein Wasserstoffatom oder eine Methylgruppe,
- X ein Chlor- oder Bromatom und
- Z eine NH-Gruppe oder ein Sauerstoffatom

bedeuten,
mit der Bedingung, daß $R_l$ eine Methylgruppe ist, wenn Z eine NH-Gruppe und X ein Chloratom sind,
sowie von pharmakologisch verträglichen Salzen derselben für die Herstellung von als Anxiolytika und Antipsychotika verwendbaren Medikamenten.

2. Verwendung nach Anspruch 1 von N-[2-(Diethylamino)ethyl)-2-methoxy-4-[(1H-4,5-dihydro-4-methyl-2-imidazolyl)amino)-5-chlorbenzamid für die Herstellung eines als Anxiolytikum und Antipsychotikum verwendbaren Medikaments.

3. Verwendung nach Anspruch 1 von N-(1-Allyl-2-pyrrolidinyl-methyl)-2-methoxy-4-[(1H-4,5-dihydro-4-methyl-2-imidazolyl)-amino]-5-chlorbenzamid für die Herstellung eines als Anxiolytikum und Antipsychotikum verwendbaren Medikaments.

4. Verwendung nach Anspruch 1 von N-[2-(Diethylamino)ethyl)-2-methoxy-4-[(1H-4,5-dihydro-4-methyl-2-imidazolyl)amino]-5-brombenzamid für die Herstellung eines als Anxiolytikum und Antipsychotikum verwendbaren Medikaments.

5. Verwendung nach Anspruch 1 von N-(1-Ethyl-2-pyrrolidinyl-methyl)-2-methoxy-4-[(1H-4,5-dihydro-4-methyl-2-imidazolyl)-amino]-5-brombenzamid für die Herstellung eines als Anxiolytikum und Antipsychotikum verwendbaren Medikaments.

6. Verwendung nach Anspruch 1 von N-[2-(Diethylamino)ethyl]-2-methoxy-4-[(1H-4,5-dihydro-2-imidazolyl)-amino]-5-brombenzamid für die Herstellung eines als Anxiolytikum und Antipsychotikum verwendbaren Medikaments.

7. Verwendung nach Anspruch 1 von N-[2-(Diethylamino)-ethyl]-2-methoxy-4-[(4,5-dihydro-2-oxazolyl)-amino]-5-chlorbenzamid für die Herstellung eines als Anxiolytikum und Antipsychotikum verwendbaren Medikaments.

8. Verwendung nach Anspruch 1 von N-(1-Ethyl-2-pyrrolidinylmethyl)-2-methoxy-4-[(1H-4,5-dihydro-4-methyl-2-imidazolyl)amino)-5-chlorbenzamid für die Herstellung eines als Anxiolytikum und Antipsychotikum verwendbaren Medikaments.

## Claims

1. Use of the compounds of formula (I)

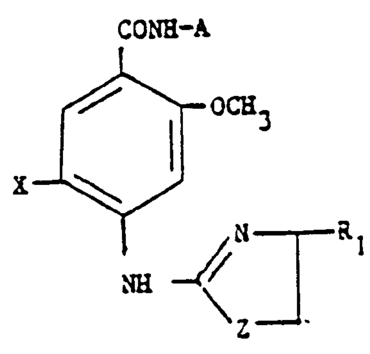

(I)

in which

– A represents a diethylamino ethyl group or a group of formula:

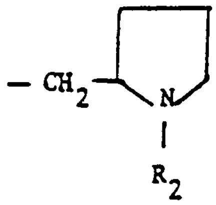

in which $R_2$ is an ethyl or allyl group,
– $R_1$ represents a hydrogen atom or a methyl group,
– X represents a chlorine or bromine atom,
– Z represents a NH group or an oxygen atom,
with the following condition:
when Z is a NH group and X a chlorine atom, $R_1$ is a methyl group,
and their pharmacologically acceptable salts, for the preparation of medicaments usable as anxiolytics and antipsychotics.

2. Use according to claim 1 of N-[2-(diethylamino)-ethyl]-2-methoxy-4-[(1H-4,5-dihydro-4-methyl-2-imidazolyl)-amino]-5-chlorobenzamide for the preparation of a medicament useful as an anxiolytic and antipsychotic.

3. Use according to claim 1 of N-(1-allyl-2-pyrrolidinylmethyl)-2-methoxy-4-[(1H-4.5-dihydro-4-methyl-2-imidazolyl)-amino]-5-chlorobenzamide for the preparation of a medicament useful as an anxiolytic and antipsychotic.

4. Use according to claim 1 of N-[2-(diethylamino)-ethyl]-2-methoxy-4-[(1H-4,5-dihydro-4-methyl-2-imidazolyl)-amino]-5-bromobenzamide for the preparation of a medicament useful as an anxiolytic and antipsychotic.

5. Use according to claim 1 of N-(1-ethyl-2-pyrrolidinylmethyl)-2-methoxy-4-[(1H-4.5-dihydro-4-methyl-2-imidazolyl)-amino]-5-bromobenzamide for the preparation of a medicament useful as an anxiolytic and antipsychotic.

6. Use according to claim 1 of N-[2-(diethylamino)-ethyl]-2-methoxy-4-[(1H-4.5-dihydro-2-imidazolyl)-amino]-5-bromobenzamide for the preparation of a medicament useful as an anxiolytic and antipsychotic.

7. Use according to claim 1 of N-[2-(diethylamino)-ethyl]-2-methoxy-4-[(4.5-dihydro-2-oxazolyl)-amino]-5-chlorobenzamide for the preparation of a medicament useful as an anxiolytic and antipsychotic.

8. Use according to claim 1 of N-(1-ethyl-2-pyrrolidinylmethyl)-2-methoxy-4-[(1H-4.5-dihydro-4-methyl-2-imidazolyl)-amino]-5-chlorobenzamide for the preparation of a medicament useful as an anxiolytic and antipsychotic.